(19) **Europäisches Patentamt / European Patent Office / Office européen des brevets**

(11) **EP 4 159 294 B1**

(12) **FASCICULE DE BREVET EUROPEEN**

(45) Date de publication et mention de la délivrance du brevet:
**12.11.2025 Bulletin 2025/46**

(21) Numéro de dépôt: **22197532.9**

(22) Date de dépôt: **23.09.2022**

(51) Classification Internationale des Brevets (IPC):
**B01D 15/18** (2006.01)    **C07C 7/13** (2006.01)
**C07C 15/08** (2006.01)

(52) Classification Coopérative des Brevets (CPC):
(C-Sets disponibles)
**B01D 15/1842; C07C 7/13**    (Cont.)

(54) **DISPOSITIF ET PROCÉDÉ DE SÉPARATION EN LIT MOBILE SIMULÉ À BRISE JET ÉTENDU**

VORRICHTUNG UND VERFAHREN ZUR TRENNUNG IN EINEM SIMULIERTEN WANDERBETT MIT ERWEITERTEM STRAHLBRECHER

SIMULATED MOVING BED SEPARATION DEVICE AND METHOD WITH EXTENDED JET BREAKER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.09.2021 FR 2110362**

(43) Date de publication de la demande:
**05.04.2023 Bulletin 2023/14**

(73) Titulaire: **IFP Energies nouvelles**
**92500 Rueil-Malmaison (FR)**

(72) Inventeurs:
• **BLANCKE, Guillaume**
  **92852 RUEIL-MALMAISON CEDEX (FR)**
• **AHMADI-MOTLAGH, Amir Hossein**
  **92852 RUEIL-MALMAISON CEDEX (FR)**
• **VONNER, Alexandre**
  **92852 RUEIL-MALMAISON CEDEX (FR)**
• **DAHRAOUI, Mohamed**
  **92500 RUEIL-MALMAISON (FR)**
• **ABOURICHA, Ayoub**
  **92500 RUEIL-MALMAISON (FR)**

(74) Mandataire: **IFP Energies nouvelles**
**Département Propriété Industrielle**
**Rond Point de l'échangeur de Solaize**
**BP3**
**69360 Solaize (FR)**

(56) Documents cités:
EP-A1- 2 138 215    WO-A1-2006/055222
WO-A2-2010/107524    FR-A1- 2 833 499
FR-A1- 2 932 999

(52) Classification Coopérative des Brevets (CPC):
(Cont.)

C-Sets
**C07C 7/13, C07C 15/08**

## Description

## Domaine Technique

L'invention se rapporte au domaine des séparations de produits naturels ou chimiques, que l'on peut difficilement séparer par distillation. On utilise alors une famille de procédés, et de dispositifs associés, connus sous le nom de procédés, ou dispositifs de séparation en lit mobile simulé, soit en contre-courant simulé, soit en co-courant simulé, que nous désignerons ci-après par l'appellation « LMS ».

Les domaines concernés sont notamment la séparation du paraxylène des autres isomères en C8 aromatiques. D'autres domaines concernés sont de façon non exclusive :

la séparation entre d'une part les paraffines normales et d'autre part les paraffines ramifiées, naphtènes, et aromatiques ; la séparation oléfines / paraffines ; la séparation du métaxylène des autres isomères en C8 aromatiques ; et la séparation de l'éthylbenzène des autres isomères en C8 aromatiques.

Spécifiquement, l'invention concerne un dispositif et procédé LMS comprenant un dispositif de distribution et de collecte de fluides au sein d'une colonne mettant en oeuvre un écoulement desdits fluides dans un milieu de particules solides, appelé lit d'adsorbant ou milieu granulaire.

Par colonne, on entend une colonne comprenant une pluralité de lits d'adsorbant disposés en série selon un sens d'écoulement du ou des fluides mis en oeuvre dans la colonne. Le fluide traversant successivement les lits d'adsorbant est appelé fluide principal pour le distinguer d'autres fluides secondaires qui peuvent être ajoutés au fluide principal par l'intermédiaire d'un dispositif de distribution et de collecte, appelé également plateau, généralement situé entre deux lits successifs.

Un plateau comprend au moins une zone de collecte et un système de vannes permettant de collecter du fluide principal et/ou d'injecter des fluides secondaires et de mélanger ces fluides secondaires avec le fluide principal. Un plateau comprend également au moins une zone de distribution qui a pour but de répartir le fluide résultant du mélange du fluide principal et des fluides secondaires sur le lit granulaire situé immédiatement en aval, au sens de l'écoulement du fluide principal. On parlera simplement dans la suite du texte de lit "aval" pour désigner le lit granulaire situé immédiatement en aval du distributeur selon la présente invention.

La présente invention concerne la zone de distribution nommée ci-après distributeur qui permet d'alimenter chaque lit granulaire, ou au moins une partie d'entre eux, à partir d'un fluide qui se présente sous la forme d'un jet issu de la zone de collecte ou du système de mélange du fluide principal et des fluides secondaires équipant le lit granulaire précédent, c'est à dire plus précisément le lit "amont", au sens de l'écoulement du fluide principal.

## Technique antérieure

De nombreux dispositifs sont connus pour distribuer, mélanger ou collecter un fluide dans une enceinte contenant des particules solides, telle en particulier qu'une colonne multi étagée. Les plateaux ont généralement comme fonctions de distribuer un fluide de manière la plus homogène possible sur la section de la colonne, de mélanger efficacement le fluide principal traversant les différents lits de la colonne avec un ou plusieurs fluides secondaires introduits au niveau de chaque lit, éventuellement de collecter un débit de fluide entre deux lits, et finalement d'homogénéiser au mieux les concentrations en sortie de lit avant l'entrée dans le lit de particules solides suivant, c'est à dire situé immédiatement en aval du dispositif considéré.

De plus, les plateaux doivent répondre à un certain nombre de contraintes telles que générer le moins possible de dispersion axiale, générer le minimum de perte de charge, et ne pas produire de perturbations hydrodynamiques pouvant altérer les performances du procédé.

Les plateaux présentent un certain nombre de caractéristiques courantes pour l'homme du métier.

Pour la bonne clarté du texte, une colonne est divisée en une pluralité de plateaux Pi et de lits d'adsorbants Ai, le plateau Pi étant disposé directement en amont du lit d'adsorbant Ai, dans le sens de l'écoulement du fluide principal. En outre, on parle de lit d'adsorbant Ai+1 pour désigner le lit d'adsorbant suivant situé en aval du lit d'adsorbant Ai, dans le sens de l'écoulement du fluide principal. De la même manière, un plateau Pi+1 désigne le plateau suivant situé en aval du plateau Pi, dans le sens de l'écoulement du fluide principal.

En outre, chaque plateau Pi de la colonne peut posséder plusieurs systèmes de vannes de collecte et d'injection et plusieurs distributeurs en liaison avec la manière dont le plateau peut être divisé en plusieurs secteurs ou régions, dénommées panneaux. Généralement, chaque panneau du plateau comporte un système de vannes de collecte et d'injection et un distributeur.

Chaque panneau peut avoir diverses formes, les plus courantes étant la division en secteurs angulaires ou en panneaux méridiens, c'est à dire en panneaux parallèles (les uns des autres), sensiblement de même largeur.

Dans chaque panneau d'un plateau Pi, on peut :

- collecter du fluide principal d'un lit d'adsorbant Ai-1 par un système dit baffle de collecte ;
- soutirer du fluide principal quittant le lit d'adsorbant Ai-1 ou mélanger du fluide principal quittant le lit d'adsorbant Ai-1 avec un fluide secondaire éventuellement injecté dans le panneau considéré par l'intermédiaire d'un réseau de distribution, se terminant par une boîte d'injection-soutirage ;

- redistribuer le fluide principal collecté seul ou en mélange avec un fluide secondaire sur le lit d'adsorbant Ai suivant par l'intermédiaire d'un distributeur.

EP0074815, US2006/0108274A1, FR2708480 fournissent des exemples de plateaux utilisés dans le cas de l'adsorption en LMS.

Dans certains cas, le lit de particules peut être bloqué par le distributeur, c'est à dire qu'il n'y a aucun espace vide entre le distributeur et le lit d'adsorbant Ai.

Dans le cas où il existe un espace vide entre le distributeur et le lit, comme décrit dans US2006/0108274A1, le distributeur peut être conçu de manière à ne pas générer de vitesses de fluide trop importantes localement en entrée de lit, afin de ne pas provoquer de fluidisation partielle du lit de particules qui peut avoir un effet négatif sur les performances du procédé. Spécifiquement, US2006/0108274A1 décrit une plaque brise-jet positionnée au-dessus du distributeur, et sous les zones ouvertes correspondantes à la sortie d'un jet de liquide, afin de limiter les fortes vitesses de ce jet en entrée du lit granulaire aval. En revanche, le distributeur tel que décrit dans US2006/0108274A1 peut ne pas être suffisant pour éliminer la fluidisation partielle du lit de particules, d'où le recours nécessaire à d'autres solutions.

Pour réduire encore la fluidisation partielle du lit de particules, l'art antérieur propose plusieurs types de solutions :

- positionner en aval du distributeur un élément de type grille ou plaque perforée permettant de limiter la turbulence et les fortes vitesses en entrée de lit de particules ;
- augmenter le nombre de panneaux et l'ouverture des baffles de collecte pour diminuer la vitesse de passage du fluide dans le dispositif de distribution/-mélange.

US2009/0321359A1 propose un distributeur comprenant les 3 éléments suivants, disposés de haut en bas en suivant le sens d'écoulement du fluide :

- un brise jet plein situé approximativement dans l'axe de l'ouverture de sortie du baffle de collecte du panneau, et centré selon l'axe dudit baffle de collecte ;
- une plaque perforée intermédiaire s'étendant latéralement au-delà du brise jet, sur une largeur comprise entre la largeur dudit brise jet, et une valeur supérieure égale à la demi largeur du panneau, à plus ou moins 5 cm près, et de degré d'ouverture compris entre 15% et 30% ; et
- une plaque de distribution s'étendant sur l'ensemble de la section du panneau P, et de degré d'ouverture compris entre 7% et 15%.

FR2932999 décrit un dispositif de distribution d'un fluide alimentant au moins un lit granulaire d'une colonne multi étagée présentant une succession de plateaux, chaque plateau soutenant un lit de solide granulaire, et étant divisé en panneaux, ledit dispositif étant appliqué à chaque panneau du plateau équipé d'un baffle de collecte situé immédiatement en amont du dispositif, ledit dispositif comprenant les 3 éléments suivants disposés dans un ordre quelconque: a) un brise jet plein situé approximativement dans l'axe de l'ouverture de sortie du baffle de collecte du panneau Pa, et centré selon l'axe dudit baffle de collecte, b) une plaque perforée intermédiaire s'étendant latéralement au delà du brise jet, d'ouverture comprise entre 10% et 40%, c) une plaque de distribution s'étendant sur l'ensemble de la section du panneau P, et de degré d'ouverture compris entre 5% et 20%.

Cependant, l'hydrodynamique des fluides à l'intérieur des lits d'adsorbants peut être améliorée.

**Résumé de l'invention**

Le problème que cherche à résoudre la présente invention est celui de l'amélioration de l'écoulement des fluides à l'intérieur d'une colonne comportant une multiplicité de lits d'adsorbant disposés en série selon le sens de l'écoulement des fluides.

La présente invention concerne un dispositif de distribution et de collecte, dénommé également ci-après panneau, permettant de collecter le fluide principal (fluide circulant dans la colonne) à partir d'un lit d'adsorbant amont et permettant d'alimenter un lit d'adsorbant aval en fluide principal. Avantageusement, le dispositif de distribution et de collecte possède en outre un système de mélange du fluide principal avec un ou plusieurs fluide(s) secondaire(s).

Selon un premier aspect, la présente invention peut être définie comme un dispositif de distribution et de collecte d'un fluide principal, le dispositif étant adapté pour alimenter un lit d'adsorbant aval d'une colonne de séparation en lit mobile simulé, le dispositif comprenant au moins un panneau, ledit panneau comprenant dans le sens de l'écoulement du fluide principal :

- une grille supérieure adaptée pour supporter un lit de particules solides d'un lit d'adsorbant amont ;
- un collecteur (ou zone de collecte) adapté pour collecter le fluide principal quittant le lit d'adsorbant amont ;
- une plaque de séparation, séparant le collecteur d'un distributeur et comprenant au moins une ouverture de sortie pour envoyer le fluide principal du collecteur vers le distributeur (ou zone de distribution) ;
- le distributeur adapté pour distribuer le fluide principal sur le lit d'adsorbant aval ; et
- une grille inférieure,

le panneau comprenant en outre :

- une boîte d'injection-soutirage adjacente à la plaque de séparation et disposée à une position sensiblement centrale du panneau, la plaque de séparation comprenant deux parties latérales situées de part et d'autre de la boîte d'injection-soutirage, chaque partie latérale s'étendant d'une largeur L de la boîte d'injection-soutirage (9) à une paroi latérale (10) du panneau (3) ;
- un élément brise-jet s'étendant perpendiculairement au sens de l'écoulement du fluide principal, l'élément brise-jet comprenant deux plaques pleines brise-jet étant :

  - étendues de part et d'autre de la boîte d'injection-soutirage ;
  - juxtaposées à la grille inférieure ;
  - disposées sous l'au moins une ouverture de sortie ;
  - adaptées pour diriger le fluide principal dans le distributeur dans une direction orthogonale au sens de l'écoulement du fluide principal,

dispositif dans lequel le rapport l/L de la largeur l de chaque plaque pleine brise-jet sur la largeur L de la partie latérale de la plaque de séparation est d'au moins 0,1.

Avantageusement, l'élément brise-jet permet notamment :

- d'améliorer l'écoulement en diminuant la dispersion axiale du fluide traversant le lit d'adsorbant aval (se rapprochant au mieux d'un écoulement piston),
- d'améliorer l'hydrodynamique des fluides à l'intérieur du lit d'adsorbant aval ; et
- de baisser la formation de sillons à la surface supérieure du lit d'adsorbant aval.

Selon un ou plusieurs modes de réalisation, l'élément brise-jet comprend un corps central disposé sous la boîte d'injection-soutirage et connectant les deux plaques pleines brise-jet.

Selon un ou plusieurs modes de réalisation, le rapport l/L de la largeur l de la plaque pleine brise-jet sur la largeur L de la partie latérale de la plaque de séparation est d'au moins 0,2, préférablement d'au moins 0,25.

Selon un ou plusieurs modes de réalisation, le rapport l/L de la largeur l de la plaque pleine brise-jet sur la largeur L de la partie latérale de la plaque de séparation est compris entre 0,1 et 0,7, préférablement entre 0,2 et 0,4, très préférablement entre 0,25 et 0,30.

Selon un ou plusieurs modes de réalisation, l'élément brise-jet et la boîte d'injection-soutirage sont juxtaposés.

Selon un ou plusieurs modes de réalisation, la distance comprise entre l'extrémité inférieure de la plaque de séparation et l'extrémité supérieure de l'élément brise-jet est inférieure à 10%, et préférablement inférieure à 6%, de la largeur du panneau.

Selon un ou plusieurs modes de réalisation, la plaque de séparation présente un degré d'ouverture compris entre 1% et 10%, et préférablement compris entre 4% et 8%.

Selon un ou plusieurs modes de réalisation, la plaque de séparation est perforée selon des trous de 5 mm à 50 mm de diamètre et/ou distants de 30 mm à 90 mm centre à centre.

Selon un deuxième aspect, la présente invention peut être définie comme un plateau de distribution et de collecte de colonne de séparation en lit mobile simulé, le plateau comprenant une pluralité de dispositifs selon le premier aspect.

Selon un troisième aspect, la présente invention peut être définie comme une colonne de séparation en lit mobile simulé, comprenant une pluralité de plateaux selon le deuxième aspect.

Selon un ou plusieurs modes de réalisation, la colonne est divisée en N lits d'adsorbant séparés par n plateaux, le nombre de lits d'adsorbant N et le nombre de plateaux n étant identiques et compris entre 4 et 24, et préférentiellement compris entre 8 et 19, très préférentiellement entre 12 et 15.

Selon un quatrième aspect, la présente invention peut être définie comme une unité de séparation en lit mobile simulé comprenant au moins une colonne selon le troisième aspect.

Selon un cinquième aspect, la présente invention peut être définie comme un procédé de séparation en lit mobile simulé, comprenant les étapes suivantes : on alimente au moins une colonne avec au moins une charge et un désorbant, et on soutire au moins un extrait et au moins un raffinat de la colonne, ladite colonne comprenant un ou plusieurs lits d'un solide adsorbant (Ai) interconnectés en boucle fermée et séparés par des plateaux (Pi) comprenant une pluralité de dispositifs selon le premier aspect, les points d'alimentation et de soutirage dans les plateaux (Pi) de la colonne étant décalés au cours du temps d'une valeur correspondant à un lit d'adsorbant avec une période de permutation et déterminant une pluralité de zones de fonctionnement de la colonne, et notamment les zones principales suivantes :

par définition, on désigne chacune des zones de fonctionnement par un numéro :

- la zone I de désorption d'un produit à séparer (e.g. paraxylène) est comprise entre l'injection du désorbant et le soutirage de l'extrait ;
- la zone II de désorption des isomères du produit à séparer est comprise entre le soutirage de l'extrait et l'injection de la charge ;
- la zone III d'adsorption du produit à séparer est comprise entre l'injection de la charge et le soutirage du raffinat ; et

- la zone IV est comprise entre le soutirage de raffinat et l'injection de désorbant ;

procédé dans lequel les lits d'adsorbant sont répartis dans les zones I à IV selon des configurations dites de type a / b / c / d, c'est-à-dire que la répartition des lits est la suivante :

- a est le nombre de lits en zone I ;
- b est le nombre de lits en zone II ;
- c est le nombre de lits en zone III ; et
- d est le nombre de lits en zone IV.

procédé dans lequel :

-

$$a = (t * 0,2) * (1 \pm 0,2) ;$$

-

$$b = (t * 0,4) * (1 \pm 0,2) ;$$

-

$$c = (t * 0,27) * (1 \pm 0,2)$$

; et

-

$$d = (t * 0,13) * (1 \pm 0,2),$$

ou

-

$$a = (t * 0,17) * (1 \pm 0,2) ;$$

-

$$b = (t * 0,42) * (1 \pm 0,2) ;$$

-

$$c = (t * 0,25) * (1 \pm 0,2) ;$$

-

$$d = (t * 0,17) * (1 \pm 0,2),$$

et

procédé dans lequel t est un nombre entier naturel compris entre 6 et 24, préférablement entre 8 et 19, très préférablement entre 12 et 15.

Selon un ou plusieurs modes de réalisation :

- la charge comprend un mélange d'aromatiques à 8 atomes de carbone ; et/ou
- le désorbant est choisi parmi le groupe constitué par un ou plusieurs isomères de diéthylbenzène et le toluène, préférablement le désorbant est le paradiéthylbenzène ou le toluène, très préférablement, le désorbant est le toluène ; et/ou
- l'adsorbant utilisé comprend ou consiste en une Faujasite choisie dans le groupe consistant en BaX, BaKX, et BaLSX.

Selon un ou plusieurs modes de réalisation :

- la température dans les lits d'adsorbant est comprise entre 140°C et 189°C, préférablement entre 155°C et 185°C, très préférablement entre 170°C et 180°C ; et/ou
- la pression dans les lits d'adsorbant est comprise entre 1 MPa et 10 MPa, préférablement entre 2 MPa et 4 MPa, très préférablement entre 2 MPa et 3 MPa ; et/ou
- la période de permutation est comprise entre 30 secondes et 100 secondes, préférablement entre 40 secondes et 80 secondes ; et/ou
- la vitesse superficielle entre les lits est comprise entre 0,2 cm/s et 2,5 cm/s et préférablement entre 0,5 cm/s et 2 cm/s.

D'autres caractéristiques et avantages de l'invention selon les aspects précités, apparaîtront à la lecture de la description ci-après et d'exemples non limitatifs de réalisations, en se référant aux figures annexées et décrites ci-après.

**Liste des figures**

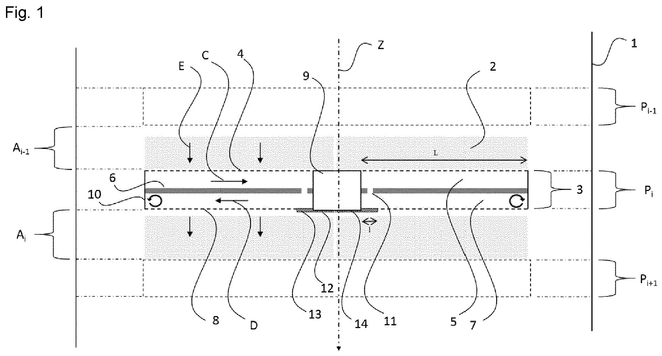

La figure 1 présente une vue en coupe partielle d'une colonne multi étagée avec 3 plateaux successifs, chaque plateau comportant une pluralité de panneaux équipés d'un système de collecte, un système de soutirage du fluide principal ou d'injection d'un fluide secondaire et d'un distributeur de référence.

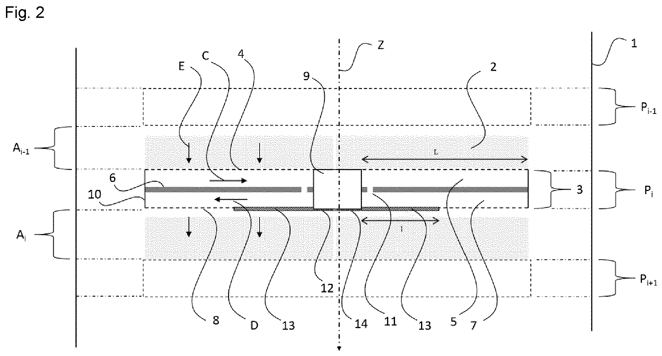

La figure 2 présente une vue en coupe partielle d'une colonne multi étagée avec 3 plateaux successifs, chaque plateau comportant une pluralité de panneaux équipés d'un système de collecte, un système de soutirage du fluide principal ou d'injection d'un fluide secondaire et d'un distributeur selon l'invention.

**Description détaillée de l'invention**

Des modes de réalisation du dispositif et du procédé selon les aspects précités vont maintenant être décrits en détail. Dans la description détaillée suivante, de nombreux détails spécifiques sont exposés afin de fournir une compréhension plus approfondie du dispositif et du procédé. Cependant, il apparaîtra à l'homme du métier que le dispositif et le procédé peuvent être mis en oeuvre sans ces détails spécifiques. Dans d'autres cas, des caractéristiques bien connues n'ont pas été décrites en détail pour éviter de compliquer inutilement la description.

Dans la présente demande, le terme « comprendre » est synonyme de (signifie la même chose que) « inclure » et « contenir », et est inclusif ou ouvert et n'exclut pas d'autres éléments non récités. Il est entendu que le terme « comprendre » inclut le terme exclusif et fermé « consister ». En outre, dans la présente description, les termes « essentiellement » ou « sensiblement » correspondent à une approximation de ± 10%, préférablement de ±5%, très préférablement de ± 2%. Par exemple, un élément disposé sensiblement à une certaine position d'un panneau, peut être disposé dans le panneau avec une approximation de ± 10%, préférablement ± 5%, par rapport à la largeur ou la hauteur du panneau.

Selon le premier aspect, l'invention peut se définir comme un dispositif de distribution et de collecte (également dénommé ci-après panneau) pour les unités de séparation LMS, le dispositif de distribution et de collecte étant adapté pour collecter un fluide en provenance d'un lit d'adsorbant amont et distribuer le fluide en direction d'un lit d'adsorbant aval.

Une unité de séparation LMS comprend au moins une colonne de séparation divisée en N lits d'adsorbant séparés par n plateaux (définissant des zones inter-lits), chaque plateau pouvant être lui-même divisé en une pluralité de panneaux. Préférablement, le nombre de lits d'adsorbant N et le nombre de plateaux n sont identiques et sont compris entre 4 et 24, et préférentiellement compris entre 8 et 19, très préférentiellement entre 12 et 15.

La division du plateau Pi en panneaux est connue de l'art antérieur. Les deux types de division les plus courants sont la division en panneaux méridiens et la division en panneaux correspondant à des secteurs angulaires. Les panneaux méridiens correspondent à des divisions du plateau Pi en éléments parallèles entre eux et contigus de manière à assurer une couverture complète de la section horizontale du plateau. Les panneaux méridiens sont orientés selon un diamètre dudit plateau, et ont préférablement sensiblement la même largeur. Selon un ou plusieurs modes de réalisation, chaque plateau est divisé en entre 4 et 24 panneaux, préférentiellement entre 12 et 16 panneaux. Préférablement les panneaux sont des panneaux méridiens.

Le dispositif

Le dispositif de distribution et de collecte comprend généralement dans le sens de l'écoulement du fluide principal,

- un collecteur adapté pour collecter le fluide principal quittant un lit d'adsorbant amont ;
- une boîte d'injection-soutirage adaptée pour extraire le fluide principal collecté ou injecter un fluide secondaire pour mélanger ledit un fluide secondaire avec le fluide principal ; et
- un distributeur adapté pour distribuer le fluide principal collecté seul ou en mélange avec un fluide secondaire sur le lit d'adsorbant aval.

En référence à la figure 1 et à la figure 2, une colonne 1 comprend une pluralité de lits de particules solides 2 disposés en série selon un sens d'écoulement E d'un fluide principal mis en œuvre dans la colonne 1. Spécifiquement, la colonne comporte selon le sens d'écoulement E du fluide principal : un plateau Pi-1, un lit d'adsorbant Ai-1 (dit lit d'adsorbant amont Ai-1), un plateau Pi, un lit d'adsorbant Ai (dit lit d'adsorbant aval Ai), et un plateau Pi+1.

En référence à la figure 1 et à la figure 2, le plateau Pi est divisé un une pluralité de panneaux 3, chaque panneau 3 comprenant de préférence des parois verticales, dont deux parois latérales 10, chaque panneau 3 comprenant en outre dans le sens de l'écoulement E du fluide principal :

- une grille supérieure 4 ou tout autre dispositif équivalent (e.g. plaque perforée) permettant de supporter le lit de particules solides 2 ;
- un collecteur 5 ou zone de collecte (représenté également par le canal de collecte C dans les figures) adapté pour collecter le fluide principal quittant le lit d'adsorbant amont Ai-1;
- une plaque de séparation 6, séparant le collecteur 5 du distributeur 7 ;
- le distributeur 7 ou zone de distribution (représenté également par le canal de distribution D dans les figures) adapté pour distribuer le fluide principal, collecté seul ou en mélange avec un fluide secondaire, sur le lit d'adsorbant aval Ai ; une grille inférieure 8 ou tout autre dispositif équivalent (e.g. plaque perforée) permettant de supporter le panneau 3 ;
- la grille supérieure 4, le collecteur 5, la plaque de séparation 6, le distributeur 7 et la grille inférieure 8 s'étendant d'une paroi latérale 10 à l'autre paroi latérale 10.

En référence à la figure 1 et à la figure 2, le panneau 3 comprend en outre une boîte d'injection-soutirage 9 adaptée pour extraire du fluide principal collecté par le collecteur 5 ou injecter un fluide secondaire pour mélanger ledit fluide secondaire avec le fluide principal. La boîte d'injection-soutirage 9 est adjacente à la plaque de séparation 6 et est disposée à une position centrale du panneau 3, i.e. située sensiblement dans l'axe central Z du panneau 3 (tel que représenté dans la vue en coupe des figures 1 et 2). L'axe central Z du panneau 3 est un axe transversal du panneau 3, i.e. un axe parallèle au sens de l'écoulement E et orthogonal au plan formé par la plaque de séparation 6.

Avantageusement, la grille supérieure 4 et la plaque de séparation 6 forment ensemble le collecteur 5 (zone de collecte) adapté pour diriger le fluide principal vers la boîte d'injection-soutirage 9.

Avantageusement, la plaque de séparation 6 comprend deux parties latérales situées de part et d'autre de la boîte d'injection-soutirage 9, i.e., la boîte d'injection-soutirage 9 sépare la plaque de séparation 6 en deux parties latérales, chaque partie latérale s'étendant d'une largeur L de la boîte d'injection-soutirage 9 à une paroi latérale 10 du panneau 3, respectivement. Il est entendu que la largeur du panneau est égale à la distance entre les deux parois latérale 10, i.e., la largeur du panneau est égale à la somme de la largeur de la boîte d'injection-soutirage 9 et des largeurs L des deux parties latérales situées de part et d'autre de la boîte d'injection-soutirage 9.

Dans la description détaillée, des caractéristiques bien connues de la boîte d'injection-soutirage 9 n'ont pas été décrites en détail pour éviter de compliquer inutilement la description. Par exemple, en référence à la figure 1 et à la figure 2, la boîte d'injection-soutirage 9 s'étend sensiblement de grille supérieure 4 à la grille inférieure 8. Cependant, il apparaîtra à l'homme du métier que la boîte d'injection-soutirage 9 puisse s'étendre sensiblement de la grille supérieure 4 à la plaque de séparation 6, ou de la plaque de séparation 6 à la grille inférieure 8. Il est également entendu que la boîte d'injection-soutirage 9 puisse être disposée entre le collecteur 5 et la plaque de séparation 6, ou entre la plaque de séparation 6 et le distributeur 7, ou entre le collecteur 5 et le distributeur 7.

Avantageusement, la plaque de séparation 6 comprend au moins une, et préférablement au moins deux, ouverture(s) de sortie 11, préférablement disposée(s) à proximité de la boîte d'injection-soutirage 9, et étant adaptée(s) pour envoyer le fluide principal du collecteur vers le distributeur 7. Préférablement, au moins une ouverture de sortie 11 est disposée de part et d'autre de la boîte d'injection-soutirage 9. En fonction du mode de fonctionnement du panneau 3, le fluide principal peut ainsi être collecté dans la boîte d'injection-soutirage 9 ou mélangé à un fluide secondaire sortant de la boîte d'injection-soutirage 9. Le fluide principal et le fluide secondaire ainsi mélangés sont redistribués vers le lit d'adsorbant aval Ai en passant dans le distributeur 7. Selon un ou plusieurs modes de réalisation, le terme à proximité correspond à une distance inférieure à 10%, préférablement inférieure à 5% de la largeur L des parties latérales de la plaque de séparation 6.

Avantageusement, la grille inférieure 8 et la plaque de séparation 6 forment ensemble le distributeur 7 (zone de distribution) pour diriger le fluide principal collecté seul ou en mélange avec un fluide secondaire vers le lit d'adsorbant aval Ai.

En référence à la figure 1 et à la figure 2, le panneau 3 comprend en outre un élément brise-jet 12 s'étendant perpendiculairement au sens de l'écoulement E du fluide principal, et étant disposé sous l'au moins une ouverture de sortie 11. Avantageusement, l'élément brise-jet 12 est adapté pour diriger le fluide principal, seul ou en mélange avec le fluide secondaire, dans le distributeur 7 dans une direction orthogonale au sens de l'écoulement E du fluide principal, i.e., vers la paroi latérale 10 du panneau 3.

Avantageusement, l'élément brise-jet 12 et la grille inférieure 8 sont accolés au sens où ils sont juxtaposés (e.g. vissés, soudés, rivetés, collés etc...) l'un à l'autre.

Avantageusement, l'élément brise-jet 12 est disposé sous la plaque de séparation 6 et sous la boîte d'injection-soutirage 9, et est disposé à une position sensiblement centrale du panneau 3, i.e. situé sensiblement dans l'axe central Z du panneau 3.

En référence à la figure 1 et à la figure 2, l'élément brise-jet 12 comprend deux plaques pleines brise-jet 13 de largeur l et s'étendant de part et d'autre de (et à partir de) la boîte d'injection-soutirage 9 vers la paroi latérale 10 du panneau 3. Selon un ou plusieurs modes de réalisation, l'élément brise-jet 12 comprend en outre un corps central 14 optionnel disposé sous la boîte d'injection-soutirage 9 connectant les deux plaques pleines brise-jet 13.

En référence à la figure 1, le rapport l/L de la largeur l de chaque plaque pleine brise-jet 13 de référence sur la largeur L d'une partie latérale de la plaque de séparation 6 est inférieur à 0,05.

En référence à la figure 2, le rapport l/L de la largeur l de chaque plaque pleine brise-jet 13 selon l'invention sur la largeur L d'une partie latérale de la plaque de séparation 6 est d'au moins 0,1, préférablement d'au moins 0,2, très préférablement d'au moins 0,25. Selon un ou plusieurs modes de réalisation, le rapport l/L est compris entre 0,1 et 0,7, préférablement entre 0,2 et 0,4, très préférablement entre 0,25 et 0,30.

Selon un ou plusieurs modes de réalisation, l'élément brise-jet 12 s'étend sur une largeur totale d'au moins 10% de la largeur du panneau 3, préférablement d'au moins 20% de la largeur du panneau 3, très préférablement d'au moins 25% de la largeur du panneau 3. Selon un ou plusieurs modes de réalisation, l'élément brise-jet 12 s'étend sur une largeur totale comprise entre 10% et 70%, préférablement entre 20% et 40%, très préférablement entre 25% et 30%, de la largeur du panneau 3.

Selon un ou plusieurs modes de réalisation, l'élément brise-jet 12 et la boîte d'injection-soutirage 9 sont accolés au sens où ils sont juxtaposés l'un à l'autre. Selon un ou plusieurs modes de réalisation, l'élément brise-jet 12, la grille inférieure et la boîte d'injection-soutirage 9 sont accolés au sens où ils sont juxtaposés les uns aux autres.

Selon un ou plusieurs modes de réalisation, la grille inférieure est une grille, par exemple de type "Johnson", (fentes étant sensiblement perpendiculaires à l'axe central du panneau). Selon un ou plusieurs modes de réalisation, la grille inférieure 8 est une plaque perforée.

Selon un ou plusieurs modes de réalisation, la distance comprise entre l'extrémité inférieure de la plaque de séparation 6 séparant le distributeur 7 du collecteur 5 et l'extrémité supérieure de l'élément brise-jet 12 est inférieure à 10%, et préférablement inférieure à 6%, de la largeur du panneau 3.

Selon un ou plusieurs modes de réalisation, la distance comprise entre l'extrémité inférieure de la plaque de séparation 6 séparant le distributeur 7 du collecteur 5 et l'extrémité supérieure de l'élément brise-jet 12 est comprise entre 1 mm et 50 mm, et préférentiellement comprise entre 5 mm et 30 mm.

Selon un ou plusieurs modes de réalisation, la plaque de séparation 6 présente un degré d'ouverture compris entre 1% et 10%, et préférentiellement compris entre 4% et 8%.

Selon un ou plusieurs modes de réalisation, la plaque de séparation 6 est perforée selon des trous de 5 mm à 50 mm de diamètre et/ou distants de 30 mm à 90 mm centre à centre.

L'invention concerne également un plateau Pi comprenant une pluralité de panneaux 3 selon l'invention.

L'invention concerne également une colonne 1 de séparation divisée en N lits d'adsorbant Ai séparées par n plateaux Pi comprenant une pluralité de panneaux 3 selon l'invention.

L'invention concerne également une unité de séparation LMS comprenant au moins une colonne 1 de séparation divisée en N lits d'adsorbant Ai séparées par n plateaux Pi comprenant une pluralité de panneaux 3 selon l'invention.

Le procédé

L'invention peut également se définir comme un procédé LMS faisant appel une unité de séparation LMS selon l'invention, dans lequel la charge à séparer est un mélange quelconque de composés, tels que des aromatiques ayant de 7 à 9 atomes de carbone, un mélange de normales et d'iso paraffines, ou un mélange de normales et d'iso oléfines.

Ainsi, l'invention concerne également un procédé de séparation LMS utilisant au moins une colonne 1 de séparation divisée en N lits d'adsorbant Ai séparées par n plateaux Pi comprenant une pluralité de panneaux 3 selon l'invention.

Dans la suite du texte, on parle d'étape pour désigner une opération ou un groupe d'opérations similaires effectuées sur un flux donné en un certain point du procédé. On décrit le procédé dans ses différentes étapes prises dans l'ordre d'écoulement des flux ou des produits.

Le procédé de séparation en LMS comprend les étapes suivantes : on alimente la colonne 1 avec au moins une charge et un désorbant, et on soutire au moins un extrait et au moins un raffinat de la colonne 1, ladite colonne 1 comprenant un ou plusieurs lits d'un solide adsorbant Ai interconnectés en boucle fermée (i.e., le dernier lit du dernier adsorbeur étant adapté pour envoyer le flux circulant dans le premier lit du premier adsorbeur) et séparés par des plateaux Pi selon l'invention, les points d'alimentation et de soutirage dans les plateaux de la colonne étant décalés au cours du temps d'une valeur correspondant à un lit d'adsorbant avec une période de permutation (notée ST) et déterminant une pluralité de zones de fonctionnement du dispositif LMS, et notamment les zones principales suivantes, désignées par définition par un numéro :

- la zone I de désorption du produit (d'intérêt) à séparer est comprise entre l'injection du désorbant et le soutirage de l'extrait ;
- la zone **II** de désorption des isomères du produit à séparer est comprise entre le soutirage de l'extrait et l'injection de la charge ;
- la zone III d'adsorption du produit à séparer est comprise entre l'injection de la charge et le soutirage du raffinat ; et
- la zone IV est comprise entre le soutirage de raffinat et l'injection de désorbant.

Selon un ou plusieurs modes de réalisation, les lits d'adsorbant sont répartis dans les zones I à IV selon des configurations dites de type a / b / c / d, c'est-à-dire que la répartition des lits est la suivante :

- a est le nombre de lits en zone I ;
- b est le nombre de lits en zone II ;
- c est le nombre de lits en zone III ; et
- d est le nombre de lits en zone IV.

Selon un ou plusieurs modes de réalisation :

-

$$a = (t * 0,2) * (1 \pm 0,2) \ ;$$

-

$$b = (t * 0,4) * (1 \pm 0,2) \ ;$$

-

$$c = (t * 0,27) * (1 \pm 0,2) \ ;$$

-

$$d = (t * 0,13) * (1 \pm 0,2),$$

et

et dans lequel t est un nombre entier naturel compris entre 6 et 24, préférablement entre 8 et 19 (e.g. entre 12 et 15).

Selon un ou plusieurs modes de réalisation :

-

$$a = (t * 0,17) * (1 \pm 0,2) \ ;$$

-

$$a = (t * 0,17) * (1 \pm 0,2) \ ;$$

-

$$b = (t * 0,42) * (1 \pm 0,2) \ ;$$

-

$$c = (t * 0,25) * (1 \pm 0,2) \ ;$$

et

-

$$d = (t * 0,17) * (1 \pm 0,2),$$

et dans lequel t est un nombre entier naturel compris entre 6 et 24, préférablement entre 8 et 19, très préférablement entre 12 et 15 (e.g. 12 ou 15).

Selon un ou plusieurs modes de réalisation, le désorbant est choisi dans le groupe constitué par un ou plusieurs isomères de diéthylbenzène et le toluène. Selon un ou plusieurs modes de réalisation, le désorbant est le paradiéthylbenzène ou le toluène. Selon un ou plusieurs modes de réalisation, le désorbant est le toluène.

Selon un ou plusieurs modes de réalisation, l'adsorbant utilisé comprend/consiste en une Faujasite choisie dans le groupe consistant en BaX, BaKX, et BaLSX.

Selon un ou plusieurs modes de réalisation, la charge est un mélange de composés essentiellement aromatiques en C8 (e.g. xylènes et éthylbenzène). Selon un ou plusieurs modes de réalisation, le mélange comprend au moins 95%, préférablement au moins 97% (e.g. au moins 99%) de composés essentiellement aromatiques en C8. Selon une ou plusieurs modes de réalisation la charge comprend au moins 15% poids de paraxylène et/ou 30% poids de métaxylène par rapport au poids total de la charge.

Un exemple de procédé de séparation LMS de grande importance industrielle concerne la séparation des coupes C8 aromatiques en vue de produire du paraxylène de pureté commerciale, typiquement à au moins 99,7% poids, et un raffinat riche en éthylbenzène, orthoxylène et métaxylène.

L'extrait produit contient du désorbant, du paraxylène et éventuellement des traces d'isomères (pureté du paraxylène supérieure à 95%, préférablement supérieur à 98%). Cet extrait peut être traité pour séparer le désorbant (e.g. par distillation) et ensuite purifié, soit par cristallisation, soit par adsorption en LMS pour augmenter la pureté du paraxylène.

Selon un ou plusieurs modes de réalisation, la température dans les lits d'adsorbant est comprise entre 140°C et 189°C et de manière préférée entre 155°C et 185°C, de manière particulièrement préférée entre 170°C et 180°C.

La pression est réglée de manière à ce que l'on reste en phase liquide en tout point du procédé selon l'invention. Selon un ou plusieurs modes de réalisation, la pression dans les lits d'adsorbant est comprise entre 1 MPa et 10 MPa, de préférence entre 2 MPa et 4 MPa, préférablement entre 2 MPa et 3 MPa.

Selon un ou plusieurs modes de réalisation, la période de permutation ST (période entre deux permutations successives des alimentations/extractions) employée est comprise entre 30 secondes et 100 secondes. Préférablement, la période de permutation ST employée est comprise entre 40 secondes et 80 secondes (e.g. 60 $\pm$10 secondes).

Selon un ou plusieurs modes de réalisation, la vitesse superficielle entre les lits est comprise entre 0,2 cm/s et 2,5 cm/s et préférentiellement entre 0,5 cm/s et 2 cm/s.

Exemples

L'efficacité du dispositif selon l'invention a été testée par simulation.

Une première simulation, dénotée exemple 1, reproduit un plateau Pi de référence tel que représenté en figure 1 alimentant un lit d'adsorbant Ai.

Une deuxième simulation, dénotée exemple 2, reproduit un plateau Pi selon l'invention tel que représenté en figure 2 alimentant un lit d'adsorbant Ai.

Dans les deux exemples, les caractéristiques structurelles des éléments du plateau Pi et du lit d'adsorbant Ai sont les suivantes :

- le diamètre du plateau Pi est de 9,7 m ;
- le plateau est divisé en panneaux méridiens ;
- la largeur du panneau méridien est de 1,15 m, la hauteur d'un lit est de 1,25 m ;
- la plaque de séparation 6 est perforée ;

- l'élément brise-jet 12 et la grille inférieure 8 sont soudés l'un à l'autre ;
- la grille inférieure 8 est perforée ;
- tous les éléments sont centrés par rapport à la boîte d'injection-soutirage 9 ;
- le lit d'adsorbant Ai est rempli d'un tamis de zéolithe de granulométrie centrée autour de 550 µm ;
- le lit d'adsorbant est rempli jusqu'à une hauteur de 30 mm sous le distributeur 7 ;
- le lit d'adsorbant Ai est alimenté en liquide à la vitesse superficielle de 1,0 cm/s ;

Dans l'exemple 1 de référence, la largeur l des plaques pleines brise-jet 13 de référence est de 3 cm.

Dans l'exemple 2 selon l'invention, la largeur l des plaques pleines brise-jet 13 selon l'invention est de 15 cm.

Les résultats sont représentés sous forme du nombre de Peclet Pe qui exprime le rapport entre la convection du fluide et la dispersion par diffusion axiale. Plus le nombre de Peclet est élevé, plus la dispersion axiale est faible.

Exemple 1 de référence : Pe = 120
Exemple 2 selon l'invention : Pe = 170

La minimisation de la dispersion axiale est bénéfique aux procédés mettant en œuvre des lits fixes de particules solides, dont en particulier les procédés d'adsorption.

**Revendications**

1. **Dispositif** de distribution et de collecte d'un fluide principal, le dispositif étant adapté pour alimenter un lit d'adsorbant aval (Ai) d'une colonne de séparation en lit mobile simulé (1), le dispositif comprenant au moins un panneau (3), ledit panneau (3) comprenant dans le sens de l'écoulement (E) du fluide principal :

- une grille supérieure (4) adaptée pour supporter un lit de particules solides (2) d'un lit d'adsorbant amont (Ai-1) ;
- un collecteur (5) adapté pour collecter le fluide principal quittant le lit d'adsorbant amont (Ai-1) ;
- une plaque de séparation (6), séparant le collecteur (5) d'un distributeur (7) et comprenant au moins une ouverture de sortie (11) pour envoyer le fluide principal du collecteur (5) vers le distributeur (7) ;
- le distributeur (7) adapté pour distribuer le fluide principal sur le lit d'adsorbant aval (Ai) ; et
- une grille inférieure (8),
le panneau comprenant en outre :

- une boîte d'injection-soutirage (9) adjacente à la plaque de séparation (6) et disposée à une position sensiblement centrale du panneau (3), la plaque de séparation (6) comprenant deux parties latérales situées de part et d'autre de la boîte d'injection-soutirage (9), chaque partie latérale s'étendant d'une largeur L de la boîte d'injection-soutirage (9) à une paroi latérale (10) du panneau (3) ;
- un élément brise-jet (12) s'étendant perpendiculairement au sens de l'écoulement (E) du fluide principal, l'élément brise-jet (12) comprenant deux plaques pleines brise-jet (13) étant :

  ○ étendues de part et d'autre de la boîte d'injection-soutirage (9) ;
  ○ juxtaposées à la grille inférieure (8) ;
  ○ disposées sous l'au moins une ouverture de sortie (11) ;
  ○ adaptées pour diriger le fluide principal dans le distributeur (7) dans une direction orthogonale au sens de l'écoulement (E) du fluide principal,

panneau dans lequel le rapport l/L de la largeur l de chaque plaque pleine brise-jet (13) sur la largeur L de la partie latérale de la plaque de séparation (6) est d'au moins 0,1.

2. Dispositif selon la revendication 1, dans lequel l'élément brise-jet (12) comprend un corps central (14) disposé sous la boîte d'injection-soutirage (9) et connectant les deux plaques pleines brise-jet (13).

3. Dispositif selon la revendication 1 ou la revendication 2, dans lequel le rapport l/L de la largeur l de la plaque pleine brise-jet (13) sur la largeur L de la partie latérale de la plaque de séparation (6) est d'au moins 0,2, préférablement d'au moins 0,25.

4. Dispositif selon l'une quelconque des revendications précédentes, dans lequel le rapport l/L de la largeur l de la plaque pleine brise-jet (13) sur la largeur L de la partie latérale de la plaque de séparation (6) est compris entre 0,1 et 0,7, préférablement entre 0,2 et 0,4, très préférablement entre 0,25 et 0,30.

5. Dispositif selon l'une quelconque des revendications précédentes, dans lequel l'élément brise-jet (12) et la boîte d'injection-soutirage (9) sont juxtaposés.

6. Dispositif selon l'une quelconque des revendications précédentes, dans lequel la distance comprise entre l'extrémité inférieure de la plaque de séparation (6) et l'extrémité supérieure de l'élément brise-jet (12) est inférieure à 10%, et préférablement inférieure à 6%, de la largeur du panneau (3) correspondant à la distance entre les deux parois latérales (10).

**7.** Dispositif selon l'une quelconque des revendications précédentes, dans lequel la plaque de séparation (6) présente un degré d'ouverture compris entre 1% et 10%, et préférablement compris entre 4% et 8%.

**8.** Dispositif selon l'une quelconque des revendications précédentes, dans lequel la plaque de séparation (6) est perforée selon des trous de 5 mm à 50 mm de diamètre et/ou distants de 30 mm à 90 mm centre à centre.

**9.** Plateau (Pi) de distribution et de collecte de colonne de séparation en lit mobile simulé (1), le plateau (Pi) comprenant une pluralité de dispositifs selon l'une quelconque des revendications 1 à 8.

**10.** Colonne (1) de séparation en lit mobile simulé, comprenant une pluralité de plateaux (Pi) selon la revendication 9.

**11.** Colonne (1) selon la revendication 10, divisée en N lits d'adsorbant (Ai) séparés par n plateaux (Pi), le nombre de lits d'adsorbant N et le nombre de plateaux n étant identiques et étant compris entre 4 et 24, et préférentiellement compris entre 8 et 19, très préférentiellement entre 12 et 15.

**12.** Unité de séparation en lit mobile simulé comprenant au moins une colonne (1) selon la revendication 10 ou la revendication 11.

**13.** Procédé de séparation en lit mobile simulé, comprenant les étapes suivantes : on alimente au moins une colonne (1) avec au moins une charge et un désorbant, et on soutire au moins un extrait et au moins un raffinat de la colonne (1), ladite colonne (1) comprenant un ou plusieurs lits d'un solide adsorbant (Ai) interconnectés en boucle fermée et séparés par des plateaux (Pi) comprenant une pluralité de dispositifs selon l'une quelconque des revendications 1 à 8, les points d'alimentation et de soutirage dans les plateaux (Pi) de la colonne (1) étant décalés au cours du temps d'une valeur correspondant à un lit d'adsorbant avec une période de permutation et déterminant une pluralité de zones de fonctionnement de la colonne (1), et notamment les zones principales suivantes désignées par définition par un numéro :

     - la zone I de désorption d'un produit à séparer est comprise entre l'injection du désorbant et le soutirage de l'extrait ;
     - la zone II de désorption des isomères du produit à séparer est comprise entre le soutirage de l'extrait et l'injection de la charge ;
     - la zone III d'adsorption du produit à séparer est comprise entre l'injection de la charge et le soutirage du raffinat ; et

     - la zone IV est comprise entre le soutirage de raffinat et l'injection de désorbant ;

procédé dans lequel les lits d'adsorbant sont répartis dans les zones I à IV selon des configurations dites de type a / b / c / d, c'est-à-dire que la répartition des lits est la suivante :

     - a est le nombre de lits en zone I ;
     - b est le nombre de lits en zone II ;
     - c est le nombre de lits en zone III ; et
     - d est le nombre de lits en zone IV.

procédé dans lequel :

-
$$a = (t * 0{,}2) * (1 \pm 0{,}2) ;$$

-
$$b = (t * 0{,}4) * (1 \pm 0{,}2) ;$$

-
$$c = (t * 0{,}27) * (1 \pm 0{,}2) ;$$

et
-
$$d = (t * 0{,}13) * (1 \pm 0{,}2),$$

ou
-
$$a = (t * 0{,}17) * (1 \pm 0{,}2) ;$$

-
$$b = (t * 0{,}42) * (1 \pm 0{,}2) ;$$

-
$$c = (t * 0{,}25) * (1 \pm 0{,}2) ;$$

et
-
$$d = (t * 0{,}17) * (1 \pm 0{,}2),$$

**14.** Procédé selon la revendication 13, comprenant au moins une des conditions opératoires suivantes :

     - La charge comprend un mélange d'aromatiques à 8 atomes de carbone ;
     - le désorbant est choisi parmi le groupe constitué par un ou plusieurs isomères de diéthylbenzène et le toluène, préférablement le désorbant est le paradiéthylbenzène ou le toluène, très préférablement, le désorbant est le toluène ;

- l'adsorbant utilisé comprend ou consiste en une Faujasite choisie dans le groupe consistant en BaX, BaKX, et BaLSX.

15. Procédé selon la revendication 13 ou la revendication 14, comprenant au moins une des conditions opératoires suivantes :

- la température dans les lits d'adsorbant est comprise entre 140°C et 189°C, préférablement entre 155°C et 185°C, très préférablement entre 170°C et 180°C ;
- la pression dans les lits d'adsorbant est comprise entre 1 MPa et 10 MPa, préférablement entre 2 MPa et 4 MPa, très préférablement entre 2 MPa et 3 MPa ;
- la période de permutation est comprise entre 30 secondes et 100 secondes, préférablement entre 40 secondes et 80 secondes ;
- la vitesse superficielle entre les lits est comprise entre 0,2 et 2,5 cm/s et préférablement entre 0,5 et 2 cm/s.

## Patentansprüche

1. Vorrichtung zum Verteilen und Sammeln eines Hauptfluids, wobei die Vorrichtung dazu ausgelegt ist, ein stromabwärts gelegenes Adsorptionsmittelbett (Ai) einer Trennkolonne mit simuliertem Fließbett (1) zu versorgen, wobei die Vorrichtung mindestens eine Platte (3) aufweist, wobei die Platte (3) in Strömungsrichtung (E) des Hauptfluids Folgendes aufweist:

- ein oberes Gitter (4), das dazu ausgelegt ist, ein Bett aus Feststoffpartikeln (2) eines stromaufwärts gelegenen Adsorptionsmittelbetts (Ai-1) zu tragen;
- einen Sammler (5), der dazu ausgelegt ist, das Hauptfluid zu sammeln, das das stromaufwärts gelegene Adsorptionsmittelbett (Ai-1) verlässt;
- eine Trennplatte (6), die den Sammler (5) von einem Sammler (7) trennt und mindestens eine Auslassöffnung (11) aufweist, um das Hauptfluid vom Sammler (5) zum Sammler (7) zu leiten;
- wobei der Sammler (7), dazu ausgelegt ist, das Hauptfluid auf das stromabwärts gelegene Adsorptionsmittelbett (Ai) zu verteilen; und
- ein unteres Gitter (8), wobei die Platte außerdem Folgendes aufweist:

    - eine Einspritz-Entnahme-Box (9), die an die Trennplatte (6) angrenzt und an einer im Wesentlichen zentralen Position der Platte (3) angeordnet ist, wobei die Trennplatte (6) zwei Seitenteile aufweist, die sich zu beiden Seiten der Einspritz-Entnahme-Box (9) befinden, wobei sich jedes Seitenteil über eine Breite L der Einspritz-Entnahme-Box (9) bis zu einer Seitenwand (10) der Platte (3) erstreckt;
- ein Strahlbrecherelement (12), das sich senkrecht zur Strömungsrichtung (E) des Hauptfluids erstreckt, wobei das Strahlbrecherelement (12) zwei massive Strahlbrecherplatten (13) aufweist, die:

    o sich zu beiden Seiten der Einspritz-Entnahme-Box (9) erstrecken;
    o neben dem unteren Gitter (8) angeordnet sind;
    o unter der mindestens einen Auslassöffnung (11) angeordnet sind;
    o dazu ausgelegt sind, das Hauptfluid im Sammler (7) in einer Richtung orthogonal zur Strömungsrichtung (E) des Hauptfluids zu leiten,

wobei bei der Platte das Verhältnis I/L der Breite I jeder massiven Strahlbrecherplatte (13) zur Breite L des Seitenteils der Trennplatte (6) mindestens 0,1 beträgt.

2. Vorrichtung nach Anspruch 1, wobei das Strahlbrecherelement (12) einen zentralen Körper (14) aufweist, der unterhalb der Einspritz-Entnahme-Box (9) angeordnet ist und die beiden massiven Strahlbrecherplatten (13) verbindet.

3. Vorrichtung nach Anspruch 1 oder Anspruch 2, wobei das Verhältnis I/L der Breite I der massiven Strahlbrecherplatte (13) zur Breite L des Seitenteils der Trennplatte (6) mindestens 0,2, vorzugsweise mindestens 0,25 beträgt.

4. Vorrichtung nach einem der vorhergehenden Ansprüche, wobei das Verhältnis I/L der Breite I der massiven Strahlbrecherplatte (13) zur Breite L des Seitenteils der Trennplatte (6) zwischen 0,1 und 0,7, vorzugsweise zwischen 0,2 und 0,4, sehr bevorzugt zwischen 0,25 und 0,30 liegt.

5. Vorrichtung nach einem der vorhergehenden Ansprüche, wobei das Strahlbrecherelement (12) und die Einspritz-Entnahme-Box (9) nebeneinander angeordnet sind.

6. Vorrichtung nach einem der vorhergehenden Ansprüche, wobei der Abstand zwischen dem unteren Ende der Trennplatte (6) und dem oberen Ende des Strahlbrecherelements (12) weniger als 10 % und vorzugsweise weniger als 6 % der Breite der Platte (3) beträgt, die dem Abstand zwischen den beiden Seitenwänden (10) entspricht.

7.  Vorrichtung nach einem der vorhergehenden Ansprüche, wobei die Trennplatte (6) einen Öffnungsgrad zwischen 1 % und 10 % und vorzugsweise zwischen 4 % und 8 % aufweist.

8.  Vorrichtung nach einem der vorhergehenden Ansprüche, wobei die Trennplatte (6) mit Löchern mit einem Durchmesser von 5 mm bis 50 mm und/oder einem Abstand von 30 mm bis 90 mm von Mitte zu Mitte perforiert ist.

9.  Verteilungs- und Sammelboden (Pi) für eine Trennkolonne mit simuliertem Fließbett (1), wobei der Boden (Pi) eine Vielzahl von Vorrichtungen gemäß einem der Ansprüche 1 bis 8 aufweist.

10. Trennkolonne (1) mit simuliertem Fließbett, die eine Vielzahl von Böden (Pi) gemäß Anspruch 9 aufweist.

11. Kolonne (1) nach Anspruch 10, unterteilt in N Adsorptionsmittelbetten (Ai), die durch n Böden (Pi) voneinander getrennt sind, wobei die Anzahl der Adsorptionsmittelbetten N und die Anzahl der Böden n identisch ist und zwischen 4 und 24, vorzugsweise zwischen 8 und 19, sehr bevorzugt zwischen 12 und 15 liegt.

12. Trenneinheit mit simuliertem Fließbett, die mindestens eine Kolonne (1) nach Anspruch 10 oder Anspruch 11 aufweist.

13. Verfahren zum Trennen in einem simulierten Fließbett, das folgende Schritte aufweist: Zuführen mindestens einer Charge und eines Desorptionsmittels zu mindestens einer Kolonne (1) und Entnehmen mindestens eines Extrakts und mindestens eines Raffinats aus der Kolonne (1), wobei die Kolonne (1) ein oder mehrere Betten aus einem adsorbierenden Feststoff (Ai) aufweist, die in einem geschlossenen Kreislauf miteinander verbunden und durch Böden (Pi) getrennt sind, die eine Vielzahl von Vorrichtungen gemäß einem der Ansprüche 1 bis 8 aufweisen, wobei die Zufuhr- und Entnahmestellen in den Böden (Pi) der Kolonne (1) im Laufe der Zeit um einen Wert versetzt werden, der einem Adsorptionsmittelbett mit einer Umschaltzeit entspricht und eine Vielzahl von Betriebszonen der Kolonne (1) bestimmt, insbesondere die folgenden Hauptzonen, die per Definition mit einer Nummer bezeichnet sind:

- die Desorptionszone I eines zu trennenden Produkts, die zwischen der Einspritzung des Desorptionsmittels und der Entnahme des Extrakts liegt;
- die Desorptionszone II der Isomere des zu trennenden Produkts, die zwischen der Entnahme des Extrakts und der Einspritzung der Charge liegt;
- die Adsorptionszone III des zu trennenden Produkts, die zwischen der Einspritzung der Charge und der Entnahme des Raffinats liegt; und
- die Zone IV, die zwischen der Entnahme des Raffinats und der Einspritzung des Desorptionsmittels liegt;

wobei im Verfahren die Adsorptionsbetten in den Zonen I bis IV gemäß den als Typ a / b / c / d bezeichneten Konfigurationen verteilt sind, d. h., dass die Verteilung der Betten wie folgt ist:

- a ist die Zahl der Betten in Zone I;
- b ist die Zahl der Betten in Zone II;
- c ist die Zahl der Betten in Zone III und
- d ist die Zahl der Betten in Zone IV.

wobei im Verfahren:

-

$$a = (t * 0{,}2) * (1 \pm 0{,}2);$$

-

$$b = (t * 0{,}4) * (1 \pm 0{,}2);$$

-

$$c = (t * 0{,}27) * (1 \pm 0{,}2)$$

und
-

$$d = (t * 0{,}13) * (1 \pm 0{,}2)$$

-

$$a = (t * 0{,}17) * (1 \pm 0{,}2);$$

oder
-

$$b = (t * 0{,}42) * (1 \pm 0{,}2);$$

-

$$c = (t * 0{,}25) * (1 \pm 0{,}2)$$

und

-

$$d = (t * 0{,}17) * (1 \pm 0{,}2),$$

wobei im Verfahren t eine natürliche Zahl zwischen 6 und 24, vorzugsweise zwischen 8 und 19, sehr bevorzugt zwischen 12 und 15 ist.

14. Verfahren nach Anspruch 13, das mindestens eine der folgenden Betriebsbedingungen aufweist:

- Die Charge beinhaltet eine Mischung aus Aromaten mit 8 Kohlenstoffatomen;
- das Desorptionsmittel wird aus der Gruppe ausgewählt, die aus einem oder mehreren Isomeren von Diethylbenzol und Toluol besteht, vorzugsweise ist das Desorptionsmittel Paradiethylbenzol oder Toluol, sehr bevorzugt ist das Desorptionsmittel Toluol;
- das verwendete Adsorptionsmittel beinhaltet oder ist ein Faujasit, das aus der Gruppe ausgewählt ist, die aus BaX, BaKX und BaLSX besteht.

15. Verfahren nach Anspruch 13 oder Anspruch 14, das mindestens eine der folgenden Betriebsbedingungen aufweist:

- die Temperatur in den Adsorptionsmittelbetten liegt zwischen 140 °C und 189 °C, vorzugsweise zwischen 155 °C und 185 °C, sehr bevorzugt zwischen 170 °C und 180 °C;
- der Druck in den Adsorptionsbetten liegt zwischen 1 MPa und 10 MPa, vorzugsweise zwischen 2 MPa und 4 MPa, sehr bevorzugt zwischen 2 MPa und 3 MPa;
- die Umschaltzeit liegt zwischen 30 Sekunden und 100 Sekunden, vorzugsweise zwischen 40 Sekunden und 80 Sekunden;
- die Oberflächengeschwindigkeit zwischen den Betten liegt zwischen 0,2 und 2,5 cm/s und vorzugsweise zwischen 0,5 und 2 cm/s.

**Claims**

1. Device for distributing and collecting a main fluid, the device being designed to feed a downstream adsorbent bed (Ai) of a simulated moving bed separation column (1), the device comprising at least one panel (3), said panel (3) comprising, in the direction of the flow (E) of the main fluid:

- an upper screen (4) designed to support a bed of solid particles (2) of an upstream adsorbent bed (Ai-1);
- a collector (5) designed to collect the main fluid leaving the upstream adsorbent bed (Ai-1);
- a separation plate (6), separating the collector (5) from a distributor (7) and comprising at least one outlet opening (11) for sending the main fluid from the collector (5) towards the distributor (7);
- the distributor (7) designed to distribute the main fluid across the downstream adsorbent bed (Ai); and
- a lower screen (8), the panel also comprising:

- an injection/withdrawal tank (9) adjacent to the separation plate (6) and disposed at a substantially central position of the panel (3), the separation plate (6) comprising two lateral parts situated on either side of the injection/withdrawal tank (9), each lateral part extending over a width L from the injection/withdrawal tank (9) to a lateral wall (10) of the panel (3);
- a jet breaker element (12) extending perpendicular to the direction of the flow (E) of the main fluid, the jet breaker element (12) comprising two solid jet breaker plates (13) that are:

o extended on either side of the injection/withdrawal tank (9);
o juxtaposed with the lower screen (8);
o disposed beneath the at least one outlet opening (11);
o designed to direct the main fluid in the distributor (7) in a direction orthogonal to the direction of the flow (E) of the main fluid,

in which panel the ratio I/L of the width I of each solid jet breaker plate (13) to the width L of the lateral part of the separation plate (6) is at least 0.1.

2. Device according to Claim 1, wherein the jet breaker element (12) comprises a central body (14) disposed beneath the injection/withdrawal tank (9) and connecting the two solid jet breaker plates (13).

3. Device according to Claim 1 or Claim 2, wherein the ratio I/L of the width I of the solid jet breaker plate (13) to the width L of the lateral part of the separation plate (6) is at least 0.2, preferably at least 0.25.

4. Device according to any one of the preceding claims, wherein the ratio I/L of the width I of the solid jet breaker plate (13) to the width L of the lateral part of the separation plate (6) is between 0.1 and 0.7, preferably between 0.2 and 0.4, very preferably between 0.25 and 0.30.

5. Device according to any one of the preceding claims, wherein the jet breaker element (12) and the injection/withdrawal tank (9) are juxtaposed.

6. Device according to any one of the preceding claims, wherein the distance between the lower end of the separation plate (6) and the upper end of the jet breaker element (12) is less than 10%, and preferably less than 6%, of the width of the panel (3) corresponding to the distance between the two lateral walls (10).

7. Device according to any one of the preceding claims, wherein the separation plate (6) has a degree of opening between 1% and 10%, and preferably between 4% and 8%.

8. Device according to any one of the preceding claims, wherein the separation plate (6) is perforated with holes 5 mm to 50 mm in diameter and/or 30 mm to 90 mm apart centre to centre.

9. Distribution and collection plate (Pi) of a simulated moving bed separation column (1), the plate (Pi) comprising a plurality of devices according to any one of Claims 1 to 8.

10. Simulated moving bed separation column (1), comprising a plurality of plates (Pi) according to Claim 9.

11. Column (1) according to Claim 10, divided into N adsorbent beds (Ai) separated by n plates (Pi), the number of adsorbent beds N and the number of plates n being identical and being between 4 and 24, and preferentially between 8 and 19, very preferentially between 12 and 15.

12. Simulated moving bed separation unit comprising at least one column (1) according to Claim 10 or Claim 11.

13. Simulated moving bed separation method, comprising the following steps: at least one column (1) is fed with at least one feedstock and a desorbent, and at least one extract and at least one raffinate are withdrawn from the column (1), said column (1) comprising one or more beds of an adsorbent solid (Ai) that are interconnected in a closed loop and separated by plates (Pi) comprising a plurality of devices according to any one of Claims 1 to 8, the feed and withdrawal points in the plates (Pi) of the column (1) being shifted over time by a value corresponding to one adsorbent bed with a switching time and determining a plurality of operating zones of the column (1), and notably the following main zones denoted by definition by a number:

- zone I for desorption of a product to be separated is between the injection of the desorbent and the withdrawal of the extract;
- zone **II** for desorption of the isomers of the product to be separated is between the withdrawal of the extract and the injection of the feedstock;
- zone **III** for adsorption of the product to be separated is between the injection of the feedstock and the withdrawal of the raffinate; and
- zone IV is between the withdrawal of raffinate and the injection of desorbent;
in which method the adsorbent beds are distributed in zones I to IV according to configurations referred to as a / b / c / d type configurations, i.e. the distribution of the beds is as follows:

- a is the number of beds in zone I;
- b is the number of beds in zone II;
- c is the number of beds in zone III; and
- d is the number of beds in zone IV.

in which method:

-

$$a = (t * 0.2) * (1 \pm 0.2);$$

-

$$b = (t * 0.4) * (1 \pm 0.2);$$

-

$$c = (t * 0.27) * (1 \pm 0.2);$$

and
-

$$d = (t * 0.13) * (1 \pm 0.2)$$

, or
-

$$a = (t * 0.17) * (1 \pm 0.2);$$

-

$$b = (t * 0.42) * (1 \pm 0.2);$$

-

$$c = (t * 0.25) * (1 \pm 0.2);$$

and
-

$$d = (t * 0.17) * (1 \pm 0.2),$$

in which method t is a natural integer between 6 and 24, preferably between 8 and 19, very preferably between 12 and 15.

14. Method according to Claim 13, comprising at least one of the following operating conditions:

- the feedstock comprises a mixture of aromatics containing 8 carbon atoms;
- the desorbent is chosen from the group made up of one or more isomers of diethylbenzene and toluene, preferably the desorbent is para-diethylbenzene or toluene, very preferably the desorbent is toluene;
- the adsorbent used comprises or consists of a faujasite chosen from the group consisting of BaX, BaKX and BaLSX.

15. Method according to Claim 13 or Claim 14, comprising at least one of the following operating conditions:

- the temperature in the adsorbent beds is between 140°C and 189°C, preferably between 155°C and 185°C, very preferably between 170°C and 180°C;
- the pressure in the adsorbent beds is between 1 MPa and 10 MPa, preferably between 2 MPa and 4 MPa, very preferably between 2 MPa and 3 MPa;
- the switching time is between 30 seconds and 100 seconds, preferably between 40 seconds and 80 seconds;
- the surface velocity between the beds is between 0.2 and 2.5 cm/s and preferably between 0.5 and 2 cm/s.

Fig. 1

Fig. 2

RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0074815 A
- US 20060108274 A1
- FR 2708480
- US 20090321359 A1
- FR 2932999